(19) Europäisches Patentamt
European Patent Office
Office européen des brevets

(11) **EP 2 931 066 B1**

(12) **EUROPEAN PATENT SPECIFICATION**

(45) Date of publication and mention
of the grant of the patent:
**05.10.2016 Bulletin 2016/40**

(21) Application number: **13802681.0**

(22) Date of filing: **12.12.2013**

(51) Int Cl.:
*A23L 35/00* (2016.01)    *A23L 29/10* (2016.01)
*A61K 9/107* (2006.01)    *A61K 8/06* (2006.01)

(86) International application number:
**PCT/EP2013/076324**

(87) International publication number:
**WO 2014/090920 (19.06.2014 Gazette 2014/25)**

(54) **EMULSIONS STABILIZED BY WHEY PROTEIN MICELLES**

MIT MOLKEPROTEINMIZELLEN STABILISIERTE EMULSIONEN

ÉMULSIONS STABILISÉES PAR DES MICELLES DE PROTÉINES DE LACTOSÉRUM

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.12.2012 EP 12196978**

(43) Date of publication of application:
**21.10.2015 Bulletin 2015/43**

(73) Proprietor: **Nestec S.A.**
**1800 Vevey (CH)**

(72) Inventors:
• **GEHIN-DELVAL, Cécile**
**F-25370 Les Hôpitaux Neufs (FR)**
• **SCHMITT, Christophe Joseph Etienne**
**CH-1077 Servion (CH)**

• **BINKS, Bernard Paul**
**Walkington**
**Yorkshire HU17 8XX (GB)**
• **DESTRIBATS, Mathieu Julien**
**F-33600 Pessac (FR)**

(74) Representative: **Couzens, Patrick John**
**Nestec S.A.**
**CT-IAM**
**Avenue Nestlé 55**
**1800 Vevey (CH)**

(56) References cited:
**EP-A1- 1 839 492      WO-A1-2007/132972
DE-A1- 19 855 767      US-A- 5 882 705
US-A1- 2010 240 608**

## Description

The present invention relates to emulsions. In particular, it relates to an emulsion comprising whey protein micelles, water and dispersed oil droplets wherein the emulsion is stabilized by the whey protein micelles. The oil droplets may have an average diameter between 40 and 1000 $\mu$m. Further aspects of the invention are a composition comprising the emulsion and a composition obtainable by drying the emulsion.

An emulsion consists of a mixture of two or more liquids that are normally immiscible. One or more liquid, the dispersed phase, is dispersed in the other, the continuous phase. In an oil-in-water emulsion for example, oil is the dispersed phase and water is the continuous phase. Emulsions are common in food, such as in mayonnaise, salad dressings, sauces, ice cream, and milk; and are frequently used in pharmaceutical, hairstyling, personal hygiene, and cosmetic products. Common emulsions are inherently unstable and thus do not form spontaneously. Energy input, such as shaking or mixing is needed to form an emulsion. Over time, emulsions tend to revert to the stable state of the phases comprising the emulsion. An example of this is seen in the separation of the oil and vinegar components of a simple vinaigrette salad dressing, an unstable emulsion that will quickly separate unless shaken almost continuously.

Emulsion stability is the ability of an emulsion to resist changes in its properties over time. Instability is mostly due to 3 phenomena: drainage, Ostwald ripening and coalescence. Drainage, or creaming, is where one of the substances migrates to the top of the emulsion due to buoyancy. Ostwald ripening is a thermodynamically-driven process due to the difference in osmotic pressure in droplets of different size. It results in the diffusion of molecules from the small droplets to the large ones through the continuous phase. Coalescence is the process in which two or more droplets merge during contact to form a single daughter droplet. Generally the three phenomena happen at the same time, leading to the instability of the emulsion and eventually the disappearance of the droplets and a return to a fully phase separated system.

Emulsifiers are substances which stabilize an emulsion by increasing its kinetic stability. One class of emulsifiers is known as "surface active substances", or surfactants. For example, adding mustard to a vinaigrette salad dressing can increase the stability of its emulsion, as chemicals in the mucilage surrounding the mustard seed hull act as emulsifiers. The emulsion will eventually disappear, but it remains longer than with oil and vinegar alone. There are a large number of emulsifiers available on the market, such as lecithins, proteins and low molecular weight emulsifiers. Particles are also known to be able to stabilize emulsions. Particle-stabilized emulsions are sometimes known as Pickering emulsions [S.U. Pickering, J. Chem. Soc. Trans., 91, 2001 (1907)].

Emulsions can vary greatly in visual appearance. Some, such as milk, appear cloudy or white due to the scattering of light as it passes through multiple phase interfaces within the emulsion. With very small emulsion droplet sizes, below about 100 nm, light is not scattered and so the emulsion appears translucent. However, for some product applications, being able to see the emulsion droplets individually provides an attractive appearance. In addition, for oil in water emulsions, having a larger emulsion droplet size reduces oxidation [K. Nikovska, Emirates Journal of Food and Agriculture, 24, 17 (2012)] and so, for food applications, reduces the risk of generating "off flavours". Emulsions with large droplets are sometimes referred to as "coarse emulsions".

Creating emulsions that have stable large droplet sizes presents a technical challenge. Emulsion droplets may increase in size due to processes such as Ostwald ripening and coalescence, but the large droplets are not stable within the emulsion and the processes continue until full phase separation results. Particles are known to provide larger droplet sizes when stabilizing emulsions than do soluble proteins or small-molecule surfactants [E. Dickinson, Trends in Food Science & Technology, 24, 4 (2012)]. Much progress has been made in the last 10-15 years concerning the better understanding of particle-stabilized emulsions both from the theoretical and practical point of view. The hydrophobicity of the particles, varied by coating them to different extents, is crucial in dictating the type (oil-in-water or water-in-oil) and coalescence stability of the emulsions. However, most of the particles used in these studies are synthetic, often inorganic materials (e.g. silica) that have limited applicability in food, pharmaceutical, agricultural or food applications. Indeed, silica particles used to stabilize emulsions are generally partially hydrophobized with adsorbed non-food grade, or even toxic, molecules or polymers. It would be desirable to develop other particles to stabilize emulsions which are of natural origin and are suitable for use in these applications. In particular it would be beneficial to identify materials to stabilize oil-in-water emulsions having large oil droplets. Emulsions have been stabilized by spores [B.P. Binks et al., Langmuir, 23, 9143 (2007)], chemically modified starch granules, cellulose particles and the water insoluble protein zein [J.W.J. de Folter et al., Soft Matter, 8, 2807 (2012)]. However, some of these materials have low nutritive value which makes them less attractive for use in food, or require additional chemical treatment to assure interfacial attachment.

The object of the present invention is to improve the state of the art and to provide an improved solution to overcome at least some of the inconveniences described above or at least to provide a useful alternative

Any reference to prior art documents in this specification is not to be considered an admission that such prior art is widely known or forms part of the common general knowledge in the field. As used in this specification, the words "comprises", "comprising", and similar words, are not to be interpreted in an exclusive or exhaustive sense. In other words, they are intended to mean "including, but not limited to".

The object of the present invention is achieved by the subject matter of the independent claims. The dependent claims further develop the idea of the present invention.

Accordingly, the present invention provides in a first aspect an emulsion comprising whey protein micelles, water and dispersed oil droplets wherein the emulsion is stabilized by the whey protein micelles and the oil droplets have an average diameter of between 40 and 1000 $\mu$m. In a second aspect, the invention relates to a composition comprising the emulsion of the invention wherein the composition is a food composition, a pharmaceutical composition, a cosmetic formulation, a nutritional formulation, a tube feeding formulation, or a drink. A further aspect of the invention is a composition obtainable by drying the emulsion.

The inventors were surprised to find that, for example, by dispersing spray dried whey protein micelles into water, adding oil and then mixing or shaking they were able to produce a stable emulsion. The inventors found that by limiting the amount of whey protein micelles it was possible to produce emulsions with large oil droplets, visible to the unaided eye. Consequently, the quantity of whey protein micelles could be used to control the resulting oil droplet size in the emulsion. The oil droplets produced were extremely stable to coalescence, remaining stable for at least 8 months. The adsorption of whey protein micelles onto the oil-water interface results in a high stability without the need to increase the viscosity of the emulsion's bulk phase. This allows more flexibility in the formulation of emulsion products with different textures, and provides smoother emulsions.

Figure 1 shows macroscopic pictures of: (a) initial dispersions at (from left to right) 0.036, 0.075, 0.132, 0.256 and 0.488 wt% whey protein micelles (WPM), (b) corresponding dispersions after pH adjustment at pH = 3 and (c) corresponding 50/50 oil/water emulsions one hour after emulsification.

Figure 2 shows macroscopic pictures of: (a) initial dispersions at (from left to right) 0.032, 0.065, 0.141, 0.276 and 0.484 wt% WPM, (b) corresponding dispersions after pH adjustment at pH = 4.7 and (c) corresponding 50/50 oil/water emulsions one hour after emulsification.

Figure 3 shows macroscopic pictures of: (a) initial dispersions at (from left to right) 0.024, 0.055, 0.082, 0.166, 0.322 and 0.662 wt.% WPM and (b) corresponding 50/50 oil/water emulsions one hour after emulsification. pH = 7.2$\pm$0.3.

Figure 4 shows optical microscopy images of emulsions at various wt.% WPM of the aqueous phase (the aqueous phase also contains 0.06M $NaN_3$) at pH=3. Pictures have been taken one hour after emulsification and samples have been gently homogenised by hand shaking before observation.

Figure 5 shows optical microscopy images of emulsions at various wt.% WPM of the aqueous phase (the aqueous phase also contains 0.06M $NaN_3$) at pH=4.7. Pictures have been taken one hour after emulsification and samples have been gently homogenised by hand shaking before observation.

Figure 6 shows optical microscopy images of emulsions at various wt.% WPM of the aqueous phase (the aqueous phase also contains 0.06M $NaN_3$) at pH=7. Pictures have been taken one hour after emulsification and samples have been gently homogenised by hand shaking before observation.

Figure 7 shows a macroscopic picture of Miglyol®-in-water emulsion stabilized by WPM at 0.024 wt.% of aqueous phase and pH 7.

Figure 8 shows a comparative evolution of the average drop diameter of oil-in-water emulsions stabilised by WPM at 0.11 wt.% of the aqueous phase as a function of the pH for two oils; Miglyol® ♦ and hexadecane ▲.

Figure 9 shows a macroscopic picture of Miglyol®-in-water emulsions with an increasing oil volume ratio, from left to right: 60, 70, 80 and 90% v/v. The pH of the aqueous phase = 4.7, WPM mass/oil mass = 60 mg/g. Picture taken 2 days after emulsification.

Figure 10 shows macroscopic pictures of Miglyol®-in-water emulsions (50/50 vol.%) with 0.4 wt.% $NaN_3$ in aqueous phase, emulsion stabilised by WPM at 0.11 wt.% of aqueous phase at different pH values (a. pH=3, b. pH=4.8 and c. pH= 7) stored for 8 months at room temperature after emulsification.

Figure 11 shows optical microscopy images of Miglyol®-in-water emulsions (50/50 vol.%) stabilised by WPM at 0.11 wt% of aqueous phase at different pH (a. pH=$_3$, b. pH=4.8 and c. pH= 7), 1 day (1) or 8 months (2) after emulsification. The aqueous phase contains 0.4 wt% $NaN_3$ and emulsions have been stored at room temperature. Samples have been gently homogenised by hand shaking before observation. Scale bar is 500 $\mu$m.

Consequently the present invention relates in part to an emulsion comprising whey protein micelles, water and dispersed oil droplets wherein the emulsion is stabilized by the whey protein micelles and the oil droplets have an average diameter of between 40 and 1000 $\mu$m. The emulsion may be stabilized by the whey protein micelles being located at the surface of the oil droplets.

"Whey protein micelles" (WPM) are defined herein as described in EP1839492A1 and as further characterized by Schmitt [C. Schmitt et al., Soft Matter, 6, 4876 (2010)], where they are referred to as whey protein microgels (WPM). Particularly, "whey protein micelles" are the micelles comprised in the whey protein micelles concentrate obtainable by the process as disclosed in EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved; whether in the concentrate form, the powder form or reconstituted from the powder, for example in water. The "whey protein micelles" are physically stable in dispersion, as a powder as well as during spray-drying or freeze-drying. Whey protein micelles can be readily tailored to different sizes or to different surface properties. Their hydrophilicity/hydrophobicity ratio can be varied by adjusting the ionic strength and pH of their environment, which can be used to optimize their adsorption at oil-water interfaces. Whey protein micelles are of natural origin, being made from milk proteins. This provides an emulsion stabilizer with good consumer acceptability.

In the present invention, "average diameter" refers to surface average diameter D[3;2]. The oil droplets in the emulsion of the current invention may be visible, as with appropriate colour contrast and lighting, individual drops are just discernable by the naked eye when they have a diameter of 40 $\mu$m. Their visibility, coupled with their stability makes the emulsion attractive and distinctive. Large, stable oil droplets provide an attractive and distinctive appearance when they occur in products such as food and cosmetics. For example, visible droplets of oil in a transparent bottle of vinaigrette salad dressing can look almost like tiny pearls or caviar.

In addition, the larger the oil droplets, the lower the surface area will be. Having a low surface area increases the chemical stability of the oil, or the stability of bioactive components within the oil. For example, large oil droplets forming an emulsion with a small overall oil surface area are less prone to oxidation. Furthermore, the release of bioactive materials from the oil droplets is slowed down by the reduced surface area and so can provide a sustained release over a longer period. For example, an energy drink having large oil droplets containing caffeine may be consumed by marathon runners. It is an advantage that the caffeine is released over a longer period of time, maintaining a constant level of caffeine in the body for longer and maximizing its bioavailability.

The emulsion of the invention may have whey protein micelles at a level between 0.01 and 5% of the emulsion, for example between 0.02 and 1% of the emulsion. Emulsion stabilizers are often one of the more expensive ingredients in an emulsion, and so it is an advantage to be able to stabilize emulsions using low levels of stabilizing material. In addition the inventors have found that using low levels of WPM to stabilize an oil-in-water emulsion leads to large oil droplets.

The emulsion of the invention may have a volume fraction of the oil between 40% and 80%, for example between 50% and 75%. The volume fraction of the oil is the volume of oil divided by the volume of all constituents of the emulsion prior to mixing. As the volume fraction of the dispersed droplets in an emulsion increases above 40% the droplet surfaces come close together and interact. This changes the rheology of the emulsion introducing viscoelastic properties which can, for example, provide attractive texture in foods. With a volume fraction of oil in the emulsion above 50% (a concentrated emulsion) the oil dispersed phase occupies more volume than the aqueous phase and the droplets become close packed. Volume fractions above 64% (the random close packing limit for equal sized spheres) are possible because not all the droplets have the same size and because droplets can deform so as not to be exact spheres. The stability of the oil droplets of the current invention at high volume fractions of oil allows the emulsion to be provided as a low moisture composition which can be easily re-dispersed in water. Low moisture compositions which are re-dispersed before use offer the advantages of having a smaller volume and weight for storage and transportation and may also be more stable against microbiological spoilage.

The oil droplets of the emulsion of the invention may contain oil soluble bioactive compounds. The greater the volume fraction of oil, the more bioactive material can be delivered. Within the scope of this invention, the term bioactive compound is understood to mean molecules or components showing biological activity or health impact when orally ingested or applied in cosmetics. The bioactive compounds may be selected from the group consisting of lipophilic carotenoids; polyphenols; vitamins, for example vitamins A and D; flavonoids; isoflavones; curcuminoids; ceramides; proanthocyanidins; terpenoids; caffeine, sterols; phytosterols; sterol-esters; tocotrienols; squalene; retinoids; and combinations thereof.

The emulsion of the invention may comprise oil selected from the group consisting of essential oils; sunflower oil; olive oil; palm oil; coconut oil, peanut oil; palm kernel oil; corn oil; hazelnut oil; sesame oil and mixtures of these. The essential oils may be the oil from a plant material selected from the group consisting of oregano, garlic, ginger, rose, mustard, cinnamon, rosemary, orange, grapefruit, lime, lemon, lemongrass, clove, clove leaf, vanilla, vanillin, mint, tea tree, thyme, grape seed, cilantro, lime, coriander, sage, eucalyptus, lavender, olive, olive leaf, anise, basil, pimento, dill, geranium, eucalyptus, aniseed, camphor, pine bark, onion, green tea, orange, artemisia herba-alba, aneth, citrus, marjoram, sage, ocimum gratissimum, thymus vulgaris, cymbopogon citratus, zingiber officinale, monodora myristica, and curcuma longa or a combination thereof. These oils are particularly suitable for use in food products, nutritional formulations or cosmetics.

The emulsion of the invention may have a pH between 2 and 8, for example between 3.5 and 7, for further example between 4 and 6. This pH range covers the values commonly encountered in foodstuffs and so it is an advantage to be able to stabilize emulsions in this pH range for culinary applications, especially emulsions with large oil droplets and high volume fractions of oil. In contrast, whey protein isolate stabilized emulsions with high volume fractions of oil are stable at neutral pH, but are very sensitive to mechanical stress at pH values between 4 and 5. Merely gently shaking the whey protein isolate emulsion at these pH values will lead to oil separation.

The oil droplets stabilized by whey protein micelles have excellent stability against coalescence. This can very useful in a commercial product. For example, once the oil droplets have been formed during the manufacture of an emulsified product such as a vinaigrette salad dressing, they remain stable during the shelf-life of the product. A consumer only has to gently shake the bottle to re-disperse the droplets before use; there is no need for vigorous shaking. The oil droplets in the emulsion of the invention may be stable against coalescence for at least 6 months, for example at least 12 months.

The aqueous phase and oil droplets of the emulsion may have contrasting colours. Contrasting colours are colours which are clearly distinguishable by eye. Colouring the different phases with contrasting colours can enhance the visual appeal of the oil droplets to a consumer. Contrasting colours may also be helpful in quality control, making the correct formation of oil droplets easy to observe. Preferably any colouring materials used should be from natural sources.

The emulsion of the invention may be comprised within a composition. The composition may be a food composition; a beverage; a beverage enhancer, for example a coffee creamer; a cosmetic composition; a pharmaceutical composition; a nutritional formulation; or a tube feeding formulation. The stability of the emulsion of the invention makes it ideal for use in foods. The size of the droplets provides an attractive organoleptic feeling. The droplets are large enough to be perceived in the mouth. As the tongue and teeth apply shear to the emulsion, the droplets are broken down, releasing oil and so providing a textural change and a burst of flavour where a flavoured oil is used. In contrast to some other particle emulsifiers, whey protein micelles are suitable for use in food. As they are derived from milk, whey protein micelles are also considered more acceptable to consumers than the synthetic chemical names of many commercial food emulsifiers. The food composition comprising the emulsion of the invention may be a dairy product; an ice-cream; a sauce; a soup; a dessert; a confectionery product; a bakery product; a salad dressing; or a pet food.

The emulsion of the invention may be used in a beverage. For example the beverage may be selected from the group consisting of bottled water-based drinks, energy drinks, milk drinks and tea beverages. Beverages with textural and visual contrast are popular with consumers, for example bubble tea. Having visible droplets may also emphasize the presence of functional ingredients to the consumer, for example when the droplets contain oil soluble bioactive compounds. The emulsion of the invention can be used to create new, interesting and attractive beverages.

Cosmetic products can benefit from the visual appeal of the emulsion and the possibility of incorporating fat soluble bioactive materials within the oil droplets. The large oil droplets provide a pleasing sensation when used in cosmetic products applied to the skin. As the emulsion is sheared across the skin, the droplets break down and release oil.

The use of whey protein micelles to stabilize an emulsion permits the formation of emulsions with a low viscosity continuous phase. This aids in the flow of the emulsion and so is an advantage for tube feeding compositions.

The stability of the oil droplets of the current invention at high volume fractions of oil allows the emulsion to be dried into a composition which can later be easily re-dispersed in water without significant coalescence of the oil droplets. Accordingly, one embodiment of the invention may be a composition obtainable, for example obtained, by drying the emulsion of the invention. Such a composition may be used to reconstitute an emulsion by dispersing it in water.

Those skilled in the art will understand that they can freely combine all features of the present invention disclosed herein. In particular, features described for the product of the present invention may be combined with the method of the present invention and vice versa. Further, features described for different embodiments of the present invention may be combined. Where known equivalents exist to specific features, such equivalents are incorporated as if specifically referred to in this specification. Further advantages and features of the present invention are apparent from the figures and non-limiting examples.

## Examples

Example 1: *Manufacture of whey protein micelle (WPM) powder*

A 50 kg batch of WPM powder was produced. This batch was obtained by heat treatment of a dispersion of whey protein isolate, WPI (Prolacta 90, Lactalis, Retiers, France) at 4 wt.% protein in softened water (160 mg.L$^{-1}$ Na$^+$) at pH 5.9 $\pm$ 0.05 (natural pH 6.48 adjusted with 1 M HCl). The WPI dispersion was pre-heated to 60 °C and then heated to 85 °C using a Soja plate-plate heat-exchanger (PHE) operating at a flow rate of 1000 L.h$^{-1}$, followed by a holding time of 15 min in a tubular heat exchanger and subsequent cooling to 4 °C. Under these operating conditions, the Reynolds number Re was approx. 1,500 ensuring a laminar flow in the PHE. More than 85% of the initial proteins were converted into WPM (determined by absorbance measurements at 278 nm after removal of the WPM by centrifugation at 26,900g for 20 min). They exhibited a hydrodynamic radius of 136 $\pm$ 7 nm and a polydispersity index of 0.1 (determined by dynamic light scattering, DLS). Thereafter, the WPM dispersion was concentrated to 22 wt.% by microfiltration using two Carbosep 0.14 membranes with a total surface of 6.8 m$^2$ (Novasep Process, Miribel, France) at a temperature of 10 °C and a flow rate of 180 L.h$^{-1}$. The liquid concentrate was then spray dried (feeding rate: 25 kg.h$^{-1}$ WPM concentrate; inlet air temperature: 145-150 °C; outlet air temperature: 75-77 °C; spraying nozzle Ø: 0.5 mm; spraying pressure 40 bar) using a GEA Niro SD6.3N spray dryer (Søborg, Denmark) and stored at 10 °C in 2 kg aluminium sealed bags. The WPM powder contained 97% of the proteins in the form of microgels. Its composition was (g/100g of wet powder): protein (Nx6.38, Kjeldhal), 91; moisture, 3.6; lactose, 3; fat, 0.4 and ash, 2. Mineral composition of the powder was (g/100g of wet powder): Ca$^{2+}$, 0.320; K$^+$, 0.409; Na$^+$, 0.468; Mg$^{2+}$, 0.060; Cl$^-$, 0.178 as determined upon HNO$_3$/H$_2$O$_2$ mineralization of the protein sample and analysis using a Vista MPX simultaneous ICP-AES spectrometer (Varian Inc. Palo Alto, CA, USA).

Example 2: *Effect of pH and whey protein micelle amount on oil in water emulsions where the oil is Miglyol 812.*

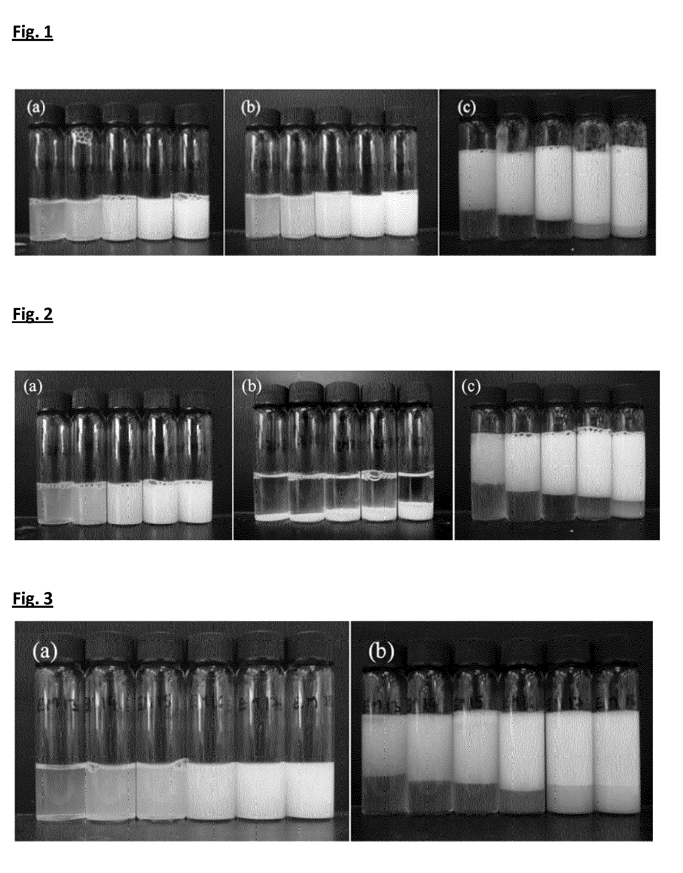

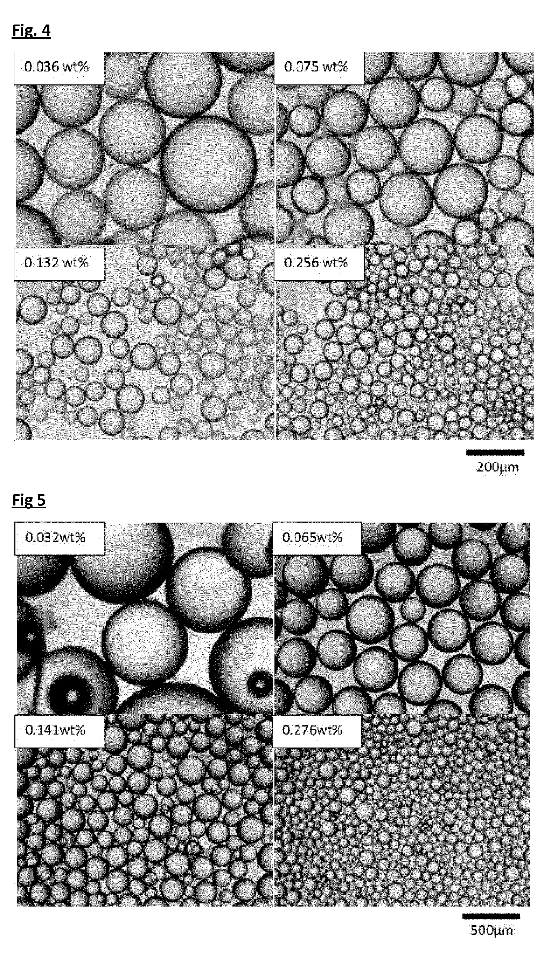

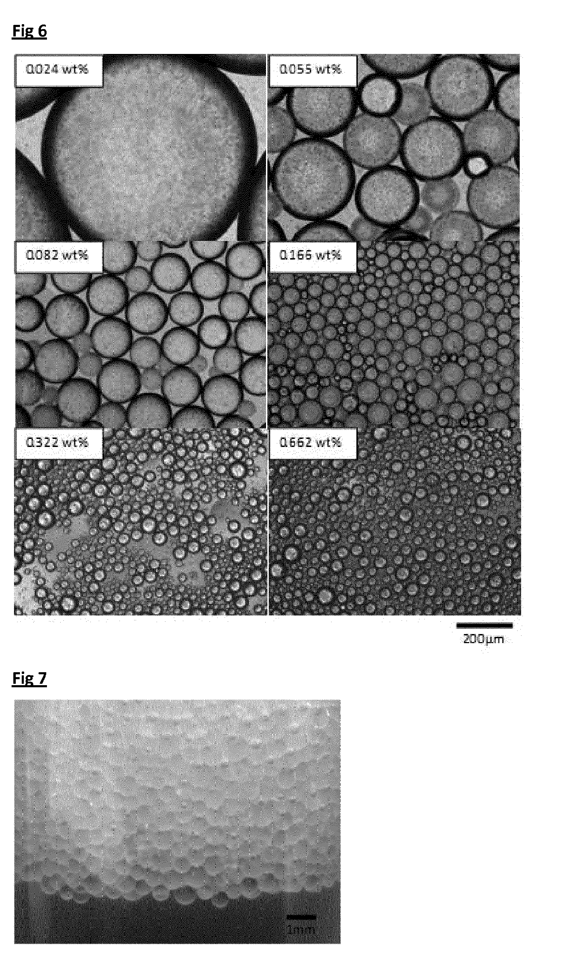

Whey protein micelle (WPM) powder as prepared in Example 1 was dispersed at 4 wt% in 10 mL Milli-Q water by means of sonication for 20 minutes with an ultrasound probe (6 mm diameter, 30% amplitude, 1 s pulse on, 0.5 s pulse off). During sonication, the dispersion was immersed into an ice bath to maintain the temperature below 50 °C. The dispersion temperature measured at the end of the process was between 40 and 45 °C). After 20 minutes, almost all the granules had been de-aggregated (just a few were still observable in the dispersion but in negligible number) leading to a particle diameter of about 215 nm and a polydispersity index below 0.07 (as determined by dynamic light scattering).
Emulsions were prepared with identical volumes of water and oil phases (50/50 vol.%) but with various wt.% of WPM between 0.024 and 0.662 wt.% initially dispersed into the aqueous phase. (This results in emulsions with whey protein micelles present at a level between 0.012 and 0.331 wt.% overall.) The oil used was Miglyol® 812 from Sasol (mixture of C8:0 and C10:0). The aqueous phases contained 0.06M NaN$_3$ as a preservative and their pH values were adjusted by addition of drops of HCl or NaOH solutions. Emulsification was done with an Ultra-Turrax mixer equipped with a small dimension head, at constant speed (13500 rpm) for 30s. Three domains of pH were probed (at pH = 3, 4.7 and 7). The emulsions were all direct (oil-in-water type) and stable. Because of the size of the drops and the density mismatch between oil and water, the emulsions form a creamed layer at the top of their containers within minutes, coexisting with a subnatent aqueous phase. Similar emulsions were formed without the addition of NaN$_3$ as a preservative, although these emulsions had to be stored at 4°C to prevent microbial growth.
Macroscopic views of the initial WPM dispersions (before and after pH adjustments) and final emulsions are shown in Figures 1, 2 and 3. In the case of dispersions at pH 7, no pH adjustment was done (native pH). Evolution of the oil drop diameter as a function of the WPM amount is shown in Figures 4, 5 and 6. In each pH domain, the drop diameter decreases as the WPM amount increases. Within this range of WPM concentration, the average drop diameter evolves from 40 to 900 $\mu$m. Figure 7 shows a macroscopic picture of the oil droplets in the emulsion at 0.012 wt.% WPM and pH 7 (0.024 wt.% WPM original dispersion in aqueous phase), demonstrating the droplets' distinctive appearance. The size distributions of the emulsion drops were determined directly by optical microscopy and the dimensions of at least 50 droplets were measured. The average drop diameter D[3;2] (surface average diameter) is estimated as defined by the following equation where $N_i$ is the total number of droplets with diameter $D_i$:

$$D[3;2] \ = \ \frac{\sum\limits_i N_i D_i^3}{\sum\limits_i N_i D_i^2}$$

Example 3: *Whey protein micelle stabilized oil-in-water emulsions where the oil is hexadecane.*

Emulsions were prepared in the same way as for Example 2, except that hexadecane (Sigma, >99%) was used as the oil phase. Direct emulsions were formed which creamed quickly.

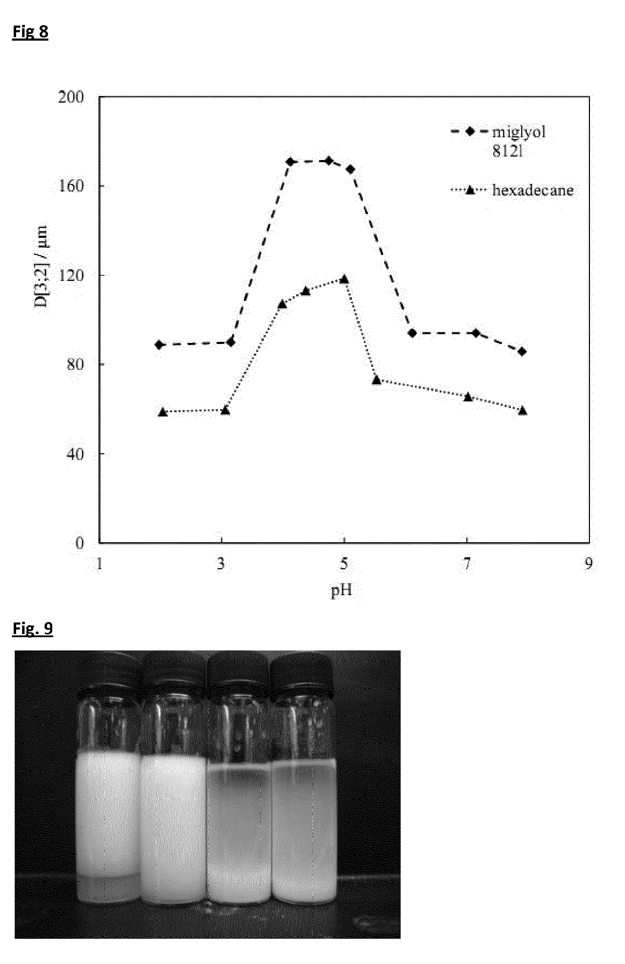

Figure 8 shows the comparative evolution of the average drop diameter of Miglyol® - in-water and hexadecane-in-water emulsions as a function of the pH. Within the range of pH = 4 to 5.5 the drops have a largest size, whereas outside this range of pH the emulsion drops are smaller and slightly flocculated. (Flocculation is the clustering of individual dispersed droplets whereby the individual droplets do not lose their identity.) The same trends in term of size evolution are observed for hexadecane and Miglyol®, although the hexadecane drops are smaller than the Miglyol® ones.

Example 4: *Influence of oil volume fraction:*

In order to study the influence of the oil volume fraction on the emulsions, different samples were prepared at pH 4.7 and 0.06 M NaN$_3$, with various Miglyol®/water volume ratios but a constant WPM mass / oil mass ratio of 60 mg/g. Higher oil volume fractions improve the stability to creaming. Emulsions below 80% v/v were found to be stable whereas emulsions above 80% v/v phase separated (Figure 9).

Example 5: *Emulsion stability:*

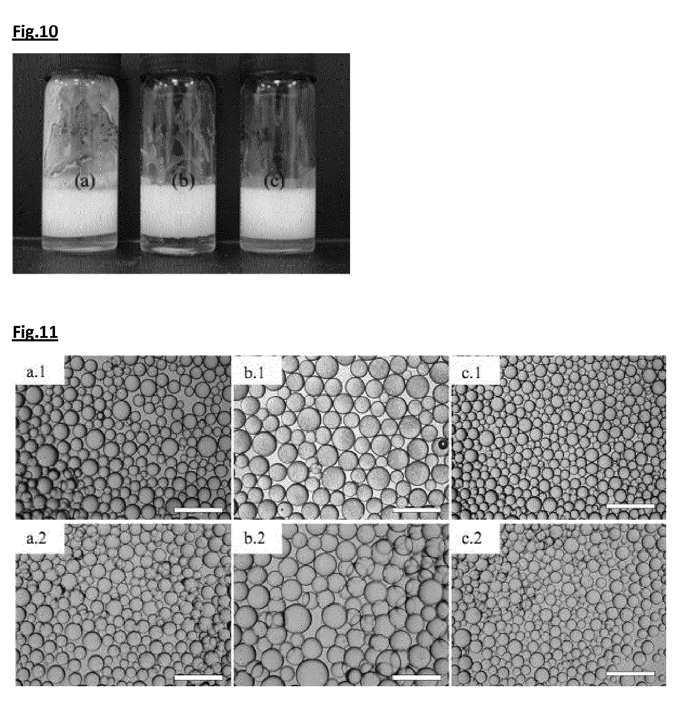

Emulsions stabilised by WPM were observed after 8 months of storage at room temperature. Figures 10 and 11 show macroscopic and microscopic observations of Miglyol®-in-water emulsions (50 vol.%) at different pH values. The aqueous phase before emulsification was a 0.11 wt% dispersion of WPM with 0.4 wt.% $NaN_3$ preservative. The size distribution of oil droplets remains almost unchanged over 8 months, demonstrating that whey protein micelles are effective at stabilizing emulsions against coalescence over an extended period.

**Claims**

1. Emulsion comprising whey protein micelles, water and dispersed oil droplets wherein the emulsion is stabilized by the whey protein micelles and the oil droplets have an average diameter of between 40 and 1000 $\mu$m.

2. An emulsion according to claim 1 wherein the whey protein micelles are present at a level between 0.01 and 5 wt.% of the emulsion.

3. An emulsion according to any one of the proceeding claims wherein the volume fraction of the oil is between 40% and 80%.

4. An emulsion according to any one of the proceeding claims wherein the oil droplets contain oil soluble bioactive compounds.

5. An emulsion according to any one of the proceeding claims wherein the oil is selected from the group consisting of essential oils; sunflower oil; olive oil; palm oil; coconut oil, peanut oil; palm kernel oil; corn oil; hazelnut oil; sesame oil and mixtures of these.

6. An emulsion according to any one of the proceeding claims wherein the pH is between 2 and 8.

7. An emulsion according to any one of the proceeding claims wherein the oil droplets are stable against coalescence for at least 6 months.

8. An emulsion according to any one of the proceeding claims wherein the aqueous phase and the oil droplets have contrasting colours.

9. Composition comprising the emulsion of any one of the proceeding claims wherein the composition is a food composition; a beverage; a beverage enhancer, for example a coffee creamer; a cosmetic composition; a pharmaceutical composition; a nutritional formulation; or a tube feeding formulation.

10. A composition according to claim 9 wherein the food composition is a dairy product; an ice-cream; a sauce; a soup; a dessert; a confectionery product; a bakery product; a salad dressing; or a pet food.

11. Composition obtainable by drying the emulsion of any one of claims 1 to 8.

12. A composition of claim 11 wherein the composition is to be dispersed in water.

**Patentansprüche**

1. Emulsion, umfassend Molkeproteinmizellen, Wasser und dispergierte Öltröpfchen, wobei die Emulsion durch die Molkeproteinmizellen stabilisiert wird und die Öltröpfchen einen durchschnittlichen Durchmesser von zwischen 40 und 1000 $\mu$m aufweisen.

2. Emulsion nach Anspruch 1, wobei die Molkeproteinmizellen in einem Niveau zwischen 0,01 und 5 Gew.-% der Emulsion vorliegen.

3. Emulsion nach einem der vorstehenden Ansprüche, wobei der Volumenanteil des Öls zwischen 40 % und 80 % liegt.

4. Emulsion nach einem der vorstehenden Ansprüche, wobei die Öltröpfchen öllösliche bioaktive Verbindungen enthalten.

5. Emulsion nach einem der vorstehenden Ansprüche, wobei das Öl ausgewählt ist aus einer Gruppe bestehend aus ätherischen Ölen; Sonnenblumenöl; Olivenöl; Palmöl; Kokosöl, Erdnussöl; Palmkernöl; Maisöl; Haselnussöl; Sesamöl und Mischungen von diesen.

6. Emulsion nach einem der vorstehenden Ansprüche, wobei der pH-Wert zwischen 2 und 8 liegt.

7. Emulsion nach einem der vorstehenden Ansprüche, wobei die Öltröpfchen mindestens 6 Monate lang koaleszenzstabil sind.

8. Emulsion nach einem der vorstehenden Ansprüche, wobei die wässrige Phase und die Öltröpfchen Kontrastfarben aufweisen.

9. Zusammensetzung, umfassend die Emulsion nach einem der vorstehenden Ansprüche, wobei die Zusammensetzung eine Nahrungsmittelzusammensetzung; ein Getränk; ein Getränkeverbesserer, zum Beispiel eine Kaffeesahne; eine kosmetische Zusammensetzung; eine pharmazeutische Zusammensetzung; eine Ernährungsformulierung; oder eine Sondennahrungsformulierung ist.

**10.** Zusammensetzung nach Anspruch 9, wobei die Nahrungsmittelzusammensetzung ein Milchprodukt; eine Eiscreme; eine Soße; eine Suppe; eine Nachspeise; eine Zuckerware; eine Backware; ein Salatdressing oder ein Haustierfutter ist.

**11.** Durch Trocknen der Emulsion nach einem der Ansprüche 1 bis 8 erhältliche Zusammensetzung.

**12.** Zusammensetzung nach Anspruch 11, wobei die Zusammensetzung in Wasser dispergiert wird.


**Revendications**

**1.** Émulsion comprenant des micelles de protéine de lactosérum, de l'eau et des gouttelettes d'huile dispersées, dans laquelle l'émulsion est stabilisée par les micelles de protéine de lactosérum et les gouttelettes d'huile ont un diamètre moyen allant de 40 à 1 000 μm.

**2.** Émulsion selon la revendication 1, dans laquelle les micelles de protéine de lactosérum sont présentes à un niveau allant de 0,01 à 5 % en poids de l'émulsion.

**3.** Émulsion selon l'une quelconque des revendications précédentes, dans laquelle la fraction volumique de l'huile va de 40 % à 80 %.

**4.** Émulsion selon l'une quelconque des revendications précédentes, dans laquelle les gouttelettes d'huile contiennent des composés bioactifs solubles dans l'huile.

**5.** Émulsion selon l'une quelconque des revendications précédentes, dans laquelle l'huile est choisie dans le groupe constitué des huiles essentielles ; de l'huile de tournesol ; de l'huile d'olive ; de l'huile de palme ; de l'huile de noix de coco, de l'huile d'arachide ; de l'huile de palmiste ; de l'huile de maïs ; de l'huile de noisette ; de l'huile de sésame et de leurs mélanges.

**6.** Émulsion selon l'une quelconque des revendications précédentes, dans laquelle le pH va de 2 à 8.

**7.** Émulsion selon l'une quelconque des revendications précédentes, dans laquelle les gouttelettes d'huile sont stables à la coalescence pendant au moins 6 mois.

**8.** Émulsion selon l'une quelconque des revendications précédentes, dans laquelle la phase aqueuse et les gouttelettes d'huile ont des couleurs contrastées.

**9.** Composition comprenant l'émulsion selon l'une quelconque des revendications précédentes, dans laquelle la composition est une composition alimentaire ; une boisson ; un exhausteur de boisson, par exemple une crème pour café ; une composition cosmétique ; une composition pharmaceutique ; une formulation nutritionnelle ; ou une formulation d'alimentation par sonde.

**10.** Composition selon la revendication 9, dans laquelle la composition alimentaire est un produit laitier ; une crème glacée ; une sauce ; une soupe ; un dessert ; un produit de confiserie ; un produit de boulangerie ; une sauce pour salade ; ou un produit alimentaire pour animal de compagnie.

**11.** Composition pouvant être obtenue par séchage de l'émulsion selon l'une quelconque des revendications 1 à 8.

**12.** Composition selon la revendication 11, dans laquelle la composition doit être dispersée dans l'eau.

**Fig. 1**

**Fig. 2**

**Fig. 3**

**Fig. 4**

**Fig 5**

**Fig 6**

**Fig 7**

Fig 8

Fig. 9

**Fig.10**

**Fig.11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1839492 A1


**Non-patent literature cited in the description**

- **S.U. PICKERING.** *J. Chem. Soc. Trans.,* 1907, vol. 91, 2001
- **K. NIKOVSKA.** *Emirates Journal of Food and Agriculture,* 2012, vol. 24, 17
- **E. DICKINSON.** *Trends in Food Science & Technology,* 2012, vol. 24 (4
- **B.P. BINKS et al.** *Langmuir,* 2007, vol. 23, 9143
- **J.W.J. DE FOLTER et al.** *Soft Matter,* 2012, vol. 8, 2807
- **C. SCHMITT et al.** *Soft Matter,* 2010, vol. 6, 4876